# EUROPEAN PATENT APPLICATION

(11) **EP 4 456 078 A1**
(43) Date of publication of application: **30.10.2024**
(21) Application number: 24171719.8
(22) Date of filing: 22.04.2024
(51) Int. Cl.: G16H 10/40

(54) **TRAINING DATA GENERATION DEVICE, TRAINING DATA GENERATION METHOD, MODEL GENERATION DEVICE, INFERENCE DEVICE, AND PROGRAM**

(30) Priority: 25.04.2023 JP 2023071603
(71) Applicant: Sintokogio, Ltd., Aichi 450-6424 (JP)
(72) Inventor: SUZUKI, Yoshihisa, Nagoya-shi, 4506424 (JP); SHIRAKI, Masataka, Nagoya-shi, 4506424 (JP); OGAERI, Saki, Nagoya-shi, 4506424 (JP); SUZUKI, Yuya, Nagoya-shi, 4506424 (JP)
(74) Representative: Bittner, Thomas L.

(57) **Abstract**

For efficiently generating training data, a main device includes a processor. The processor performs: a wear determination process of determining, in view of an output signal from a sub-sensor provided close to an odor sensor associated with a service user, whether the odor sensor is worn by the service user; a first acquisition process of acquiring an output signal from the odor sensor; a second acquisition process of acquiring type information indicative of a type of excretion of the service user, which has been determined by a service provider; and a training data generation process of generating training data which includes sensor information indicative of the output signal having been acquired in the first acquisition process from the odor sensor that has been, in the wear determination process, determined to be worn by the service user and which includes the type information having been acquired in the second acquisition process. (Fig. 2)

## Description

The present invention relates to an inference device and an inference method each of which infers a type of excretion of a service user. The present invention also relates to a model generation device and a model generation method each of which generates a model used for such inference, through machine learning. The present invention also relates to a training data generation device and a training data generation method each of which generates training data used for such machine learning.

### Background Art

In facilities, the purpose of which is to provide care services and/or medical services, it is necessary that service providers be informed of excretion of service users on a timely basis. However, in such a facility, some service users (including service users sleeping at night) are incapable of or have difficulty in notifying the service providers of the excretion. In order to be informed of the excretion of such service users, the service providers are necessary to take undergarments of the service users off and check whether or not the service users have excreted. This is a heavy burden on the service providers.

In order to reduce such a burden, it is demanded that information processing technology is used in the caring and medical practice. For example, document JP H09-43182 A discloses such information processing technology usable in the caring and medical practice. Document JP H09-43182 A discloses a biological monitoring device for monitoring a health condition of a patient in view of an output signal from a gas sensor attached to a T-bandage.

### Summary of invention

### Technical problem

In facilities, the purpose of which is to provide care services and/or medical services, a technique is demanded of notifying the service providers of types of excretion of the service users as well as of whether the service users have excreted. This is because the service providers are necessary to take different action depending on the type of the excretion. For example, in a case where the type of the excretion is flatus, it is unnecessary that the service providers take any measures, whereas, in a case where the type of the excretion is a stool, it is necessary that the service providers take measures. In this case, it is possible that the types of the excretion of the service users are determined by a model generated through machine learning (learned model). However, a technique has not been established of efficiently generating training data necessary for generating a model through machine learning, in particular, through supervised learning.

An aspect of the present invention is achieved in light of the foregoing problem. It is an object of the aspect of the present invention to achieve a technique that enables efficient generation of training data. An object of another aspect of the present invention is to achieve a technique of generating a model with use of the training data so generated. An object of still another aspect of the present invention is to achieve a technique of inferring a type of excretion with use of such a model.

### Solution to Problem

A training data generation device in accordance with an aspect of the present invention includes at least one processor, the at least one processor being configured to carry out: a wear determination process of determining, in view of an output signal from a sub-sensor provided close to an odor sensor associated with a service user, whether or not the odor sensor is worn by the service user; a first acquisition process of acquiring an output signal from the odor sensor; a second acquisition process of acquiring type information indicative of a type of excretion of the service user, the type having being determined by a service provider; and a training data generation process of generating training data which includes sensor information indicative of the output signal having been acquired in the first acquisition process from the odor sensor that has been, in the wear determination process, determined to be worn by the service user and which includes the type information having been acquired in the second acquisition process.

A training data generation method in accordance with an aspect of the present invention includes: carrying out, with use of at least one processor, a wear determination process of determining, in view of an output signal from a sub-sensor provided close to an odor sensor associated with a service user, whether or not the odor sensor is worn by the service user; carrying out, with use of the at least one processor, a first acquisition process of acquiring an output signal from the odor sensor; carrying out, with use of the at least one processor, a second acquisition process of acquiring type information indicative of a type of excretion of the service user, the type having being determined by a service provider; and carrying out, with use of the at least one processor, a training data generation process of generating training data which includes sensor information indicative of the output signal having been acquired in the first acquisition process from the odor sensor that has been, in the wear determination process, determined to be worn by the service user and which includes the type information having been acquired in the second acquisition process.

A model generation device in accordance with an aspect of the present invention includes at least one processor, the at least one processor being configured to carry out a model generation process of generating, through machine learning using training data generated by the training data generation device in accordance with an aspect, a model into which sensor information indicative of an output signal from an odor sensor is to be inputted and from which type information indicative of a type of excretion is to be outputted.

An inference device in accordance with an aspect of the present invention includes at least one processor, the at least one processor being configured to carry out an inference process of inferring, with use of a model generated by the model generation device in accordance with an aspect of the present invention, a type of excretion of a service user in view of an output signal from an odor sensor associated with the service user.

The training data generation device, the model generation device, and the inference device each in accordance with an aspect of the present invention may be each achieved by a computer. In this case, the scope of the present invention also encompasses a device control program for causing the computer to operate as components (software elements) included in each of the devices, so as to achieve each of the devices with use of the computer, and a computer-readable storage medium storing the program.

### Advantageous effects of invention

According to an aspect of the present invention, it is possible to efficiently generate training data. Further, according to an aspect of the present invention, it is possible to generate a model with use of the training data so generated. Furthermore, according to an aspect of the present invention, it is possible to infer a type of excretion with use of such a model.

### Brief description of drawings

- Fig. 1: is a view schematically illustrating a configuration of a notification system in accordance with an embodiment of the present invention.
- Fig. 2: is a block diagram illustrating a configuration of a main device included in the
- Fig. 3: notification system illustrated in Fig. 1. is a view illustrating outlines of a training data generation method, a model generation method, and an inference method each in accordance with an aspect of the present invention.
- Fig. 4: is a flowchart illustrating an operation (including the training data generation method illustrated in Fig. 3) of the notification system illustrated in Fig. 1 in a tentative operation period.
- Fig. 5: is a view illustrating examples of a notification screen and an input screen which are to be displayed in the tentative operation period on a notification terminal.
- Fig. 6: is a flowchart illustrating a flow of the model generation method illustrated in Fig. 3.
- Fig. 7: is a flowchart illustrating an operation (including the inference method illustrated in Fig. 3) of the notification system illustrated in Fig. 1 in an actual operation period.
- Fig. 8: is a view illustrating an example of a notification screen which is to be displayed in the actual operation period on the notification terminal.

### Description of embodiments

### (Configuration of Notification System)

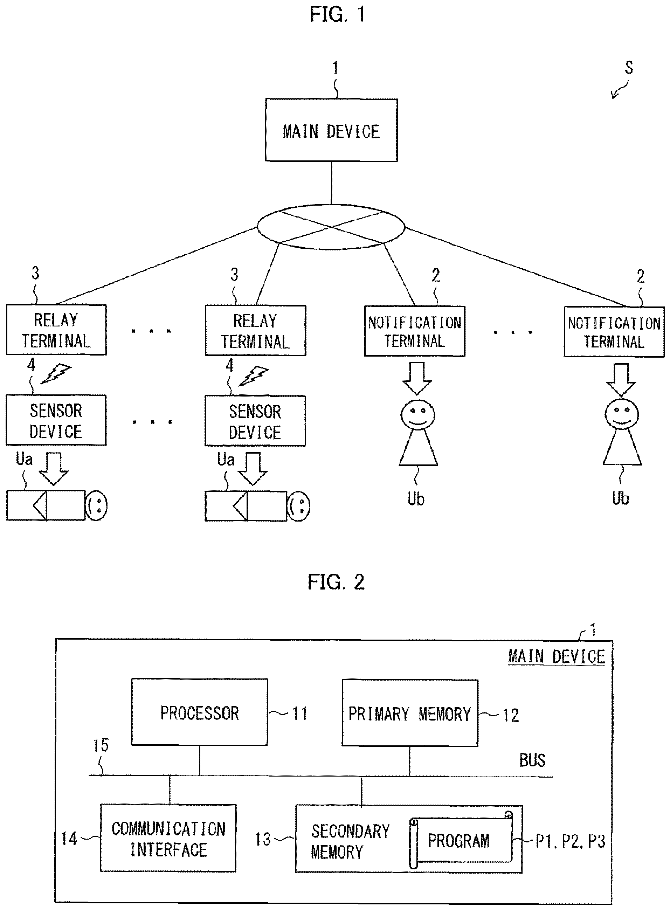

With reference to Fig. 1, the following description will discuss a notification system S in accordance with an embodiment of the present invention. Fig. 1 is a view schematically illustrating a configuration of the notification system S.

The notification system S notifies service providers Ub of excretion of each of service users Ua in a facility, the purpose of which is to provide care services and/or medical services.

As illustrated in Fig. 1, the notification system S includes a main device 1, a plurality of notification terminals 2, a plurality of relay terminals 3, and a plurality of sensor devices 4. The main device 1 communicates with the notification terminals 2 on a one-to-multiple basis via a network. The main device 1 communicates with the relay terminals 3 on a one-to-multiple basis via a network. Each of the relay terminals 3 communicates with the corresponding one of the sensor devices 4 on a one-to-one basis without using a network (for example, with use of near field communication).

The sensor devices 4 are worn by the service users Ua. For example, the sensor devices 4 are worn inside the undergarments (such as diapers or underwear) of the service users Ua.

Each of the sensor devices 4 includes an odor sensor (main sensor) associated with the service user Ua and a sub-sensor provided close to the odor sensor. The sensor device 4 transmit, to the relay terminal 3, output signals from the odor sensor and the sub-sensor together with an ID (identification information) of the service user Ua. In the present embodiment, as the sub-sensor, a temperature sensor is used.

Each of the relay terminals 3 is provided close to the corresponding one of the sensor devices 4. For example, the relay terminal 3 is provided in a room in which the service user Ua is present. The relay terminal 3 receives, from the sensor device 4, the output signals from the odor sensor and the temperature sensor together with the ID of the service user Ua. In addition, the relay terminal 3 transmits, to the main device 1, the output signals from the odor sensor and the temperature sensor that have been received from the sensor device 4, together with the ID of the service user Ua. In the present embodiment, as the relay terminal 3, a smartphone is used. Alternatively, a stationary wireless relay device may be used as the relay terminal 3.

The main device 1 is provided outside the above-described facility. For example, the main device 1 is a workstation provided in a data center. The main device 1 receives, from the relay terminal 3, the output signals from the odor sensor and the temperature sensor together with the ID of the service user Ua. The main device 1 detects the excretion of the service user Ua with reference to the output signal from the odor sensor that has been received from the relay terminal 3. Subsequently, (i) in a training data generation method M1 described later, the main device 1 transmits data indicative of a notification screen σ1a and an input screen σ1b, and (ii) in an inference method M3 described later, the main device 1 transmits data indicative of a notification screen σ2. Note that, for example, a personal computer provided in the facility may be alternatively used as the main device 1.

The notification terminals 2 are carried by the service providers Ub. The notification terminal 2 (i) receives, in the training data generation method M1 described later, the data indicative of the notification screen σ1a and the input screen σ1b from the main device 1 and (ii) receives, in the inference method M3 described later, the data indicative of the notification screen o2 from the main device 1. The notification terminal 2 includes a display of a touch panel type. On the display, the notification terminal 2 (i) displays, in the training data generation method M 1 described later, the notification screen σ1a and the input screen σ1b based on the data acquired from the main device 1 and (ii) displays, in the inference method M3 described later, the notification screen σ2 based on the data acquired from the main device 1. In the present embodiment, as the notification terminal 2, a smartphone is used. Note that, in the following descriptions, transmitting data indicative of each screen is described also as transmitting the screen, and receiving data indicative of each screen is described also as receiving the screen.

### (Configuration of Main Device)

With reference to Fig. 2, the following will describe the configuration of the main device 1 included in the notification system S. Fig. 2 is a block diagram illustrating the configuration of the main device 1.

As illustrated in Fig. 2, the main device 1 includes a processor 11, a primary memory 12, a secondary memory 13, a communication interface 14, and a bus 15. The processor 11, the primary memory 12, the secondary memory 13, and the communication interface 14 are connected with each other via the bus 15. Examples of a device usable as the main device 1 include a workstation constituting a cloud server.

The secondary memory 13 stores a training data generation program P1, a model generation program P2, and an inference program P3. The processor 11 loads, on the primary memory 12, the training data generation program P1 stored in the secondary memory 13. The processor 11 then carries out processes included in the training data generation method M1 (described later) in accordance with instructions included in the training data generation program P1 loaded on the primary memory 12. The secondary memory 13 also stores training data D generated by the training data generation method M1. The processor 11 loads, on the primary memory 12, the model generation program P2 stored in the secondary memory 13. The processor 11 then carries out processes included in the model generation method M2 (described later) in accordance with instructions included in the model generation program P2 loaded on the primary memory 12. The secondary memory 13 also stores a model M generated by the model generation method M2. The processor 11 loads, on the primary memory 12, the inference program P3 stored in the secondary memory 13. The processor 11 then carries out processes included in the inference method M3 (described later) in accordance with instructions included in the inference program P3 loaded on the primary memory 12.

Examples of a device usable as the processor 11 includes a central processing unit (CPU).

Examples of a device usable as the primary memory 12 include a semiconductor random access memory (RAM). Examples of a device usable as the secondary memory 13 include a hard disk drive (HDD).

The communication interface 14 is an interface for communicating with the notification terminals 2 and the relay terminals 3 via a network. Examples of an interface usable as the communication interface 14 include an Ethernet (registered trademark) interface. Examples of a usable network include a personal area network (PAN), a local area network (LAN), a campus area network (CAN), a metropolitan area network (MAN), a wide area network (WAN), a global area network (GAN), and an internetwork containing a combination thereof. The internetwork may be an intranet, or may be an extranet, or may be the Internet.

Note that the training data generation program P1 for causing the processor 11 to carry out the training data generation method M1 may be stored in a computer-readable non-transitory tangible storage medium. This storage medium can be the secondary memory 13 or another storage medium. For example, a tape, a disk, a card, a semiconductor memory, a programmable logic circuit, or the like can be used as said another storage medium. The same applies to the model generation program P2 and the inference program P3.

The present embodiment employs a configuration in which a single processor (the processor 11) is used to carry out the training data generation method M1. Note, however, that the present invention is not limited to this. That is, it is alternatively possible to employ a configuration in which a plurality of processors are used to carry out the training data generation method M1. In this case, the plurality of processors for carrying out the training data generation method M1 may be provided in a single computer and be configured to be communicable with each other via a bus or may be dispersedly provided in a respective plurality of computers and be configured to be communicable with each other via a network. For example, the following alternative aspects are also possible: an aspect in which processors included in a respective plurality of computers constituting a cloud server work together to carry out the training data generation method M1; and an aspect in which the processor 11 of the main device 1 and processors of the notification terminals 2 work together to carry out the training data generation method M1. The same applies to the model generation method M2 and the inference method M3.

The present embodiment employs a configuration in which each of the training data generation method M1, the model generation method M2, and the inference method M3 is carried out by the main device 1. Note, however, that the present invention is not limited to this. The scope of the present invention also encompasses each of a training data generation device for carrying out at least the training data generation method M1, a model generation device for carrying out at least the model generation method M2, and an inference device for carrying out at least the inference method M3. Here, the training data generation device only needs to carry out the training data generation method M1, and may or may not carry out the model generation method M2 and the inference method M3. The model generation device only needs to carry out the model generation method M2, and may or may not carry out the training data generation method M1 and the inference method M3. The inference device only needs to carry out the inference method M3, and may or may not carry out the training data generation method M1 and the model generation method M2.

### (Outlines of Training Data Generation Method, Model Generation Method, and Inference Method)

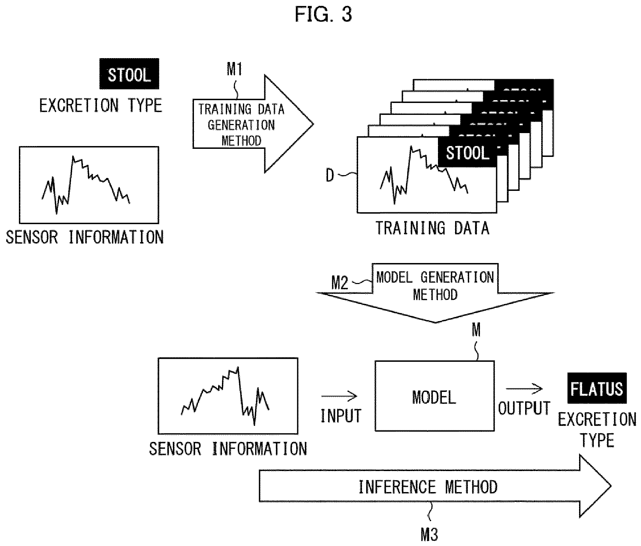

With reference to Fig. 3, the following description will discuss the outlines of the training data generation method M1, the model generation method M2, and the inference method M3. Fig. 3 is a view schematically illustrating the outlines of the training data generation method M1, the model generation method M2, and the inference method M3.

The training data generation method M1 is a method for generating training data D including (i) sensor information indicative of an output signal from the odor sensor that is associated with each of the service users Ua and that has been determined to be worn by the service user Ua and (ii) type information indicative of a type of the excretion of the service user Ua. The type information can be regarded as a label attached to the sensor information.

Here, the sensor information may be, for example, numerical data indicative of an output signal from the odor sensor. Alternatively, the sensor information may be image data including a graph indicative of an output signal from the odor sensor. In the present embodiment, as the sensor information, image data including a graph indicative of an output signal from the odor sensor is used. The type information may be a string (such as "stool", "urine", and "flatus") indicative of a type of the excretion. Alternatively, the type information may be a symbol (such as C1, C2, and C3) indicative of a type of the excretion. In the present embodiment, as the type information, a symbol indicative of at least one of the types of the excretion, which are a stool, urine, and flatus is used. A single piece of the type information may be indicative of a plurality of types, e.g., both a stool and urine.

The model generation method M2 is a method for generating, through supervised learning, the model M into which the sensor information indicative of the output signal from the odor sensor is to be inputted and from which the type information indicative of the type of the excretion is to be outputted. In the present embodiment, as the supervised learning, machine learning using training data D generated by the training data generation method M1 is employed.

Here, the model M may be any model that can be applied to a classification problem and that can be generated through supervised learning. For example, the model M may be implemented by a k-nearest neighbor, a decision tree (classification tree), a random forest, a support-vector machine, or a neural network. In the present embodiment, as the model M, a neural network is used.

The inference method M3 is a method for inferring, with use of the model generated through supervised learning, a type of excretion of each of the service users Ua in view of an output signal from the odor sensor associated with the service user Ua. In the present embodiment, as the model generated through the supervised learning, the model M generated by the model generation method M2 is used.

The operations performed by the main device 1 differ between a tentative operation period and an actual operation period. That is, in the tentative operation period, the main device 1 notifies each of the service providers Ub of the excretion of each of the service users Ua without the type specified, whereas, in the actual operation period, the main device 1 notifies each of the service providers Ub of the excretion of each of the service users Ua with the type specified.

In the present embodiment, the training data generation method M1 is incorporated into the operation performed by the main device 1 during the tentative operation period. That is, the training data D is generated during the tentative operation period. The model generation method M2 is carried out by the main device 1 after the end of the tentative operation period and before a start of the actual operation period. That is, the model M is generated after the end of the tentative operation period and before the start of the actual operation period. The inference method M3 is incorporated into the operation performed by the main device 1 during the actual operation period. That is, the model M is used during the actual operation period.

The following descriptions will discuss (i) the operation (including the training data generation method M1) performed by the main device 1 during the tentative operation period, (ii) the model generation method M2, and (iii) the operation (including the inference method M3) performed by the main device 1 during the actual operation period.

### (Operation of Main Device during Tentative Operation Period)

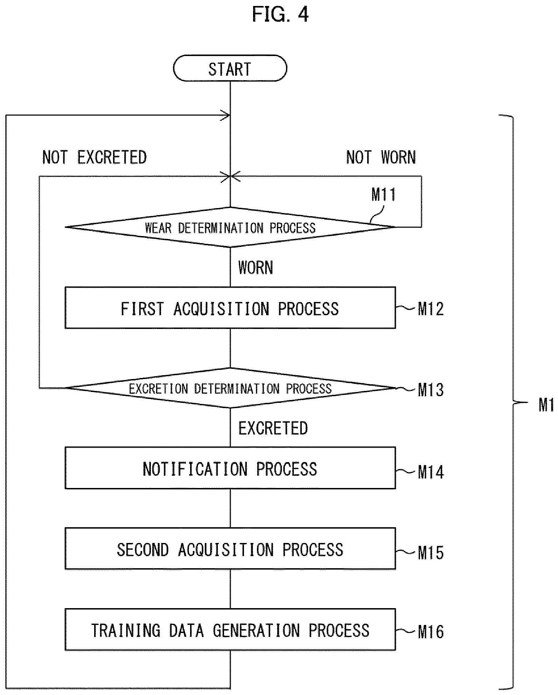

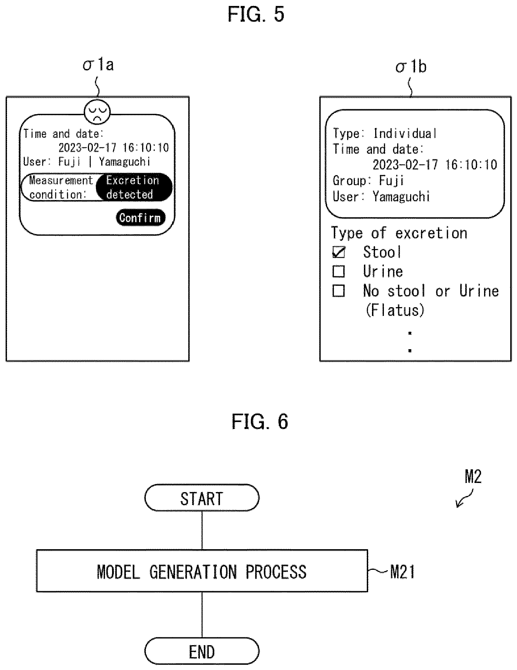

With reference to Figs. 4 and 5, the following description will discuss the operation performed by the main device 1 during the tentative operation period. Fig. 4 is a flowchart illustrating a flow of the operation performed by the main device 1 during the tentative operation period. Fig. 5 is a view illustrating examples of the notification screen o1a and the input screen o1b which are to be displayed on the notification terminals 2 during the tentative operation period.

In the tentative operation period, the main device 1 carries out the wear determination process M11, a first acquisition process M12, an excretion determination process M13, a notification process M14, a second acquisition process M15, and a training data generation process M16 that are shown in Fig. 4. The training data generation method M1 includes the wear determination process M11, the first acquisition process M12, the excretion determination process M13, the notification process M14, the second acquisition process M15, and the training data generation process M16.

The wear determination process M11 is a process of determining whether or not the odor sensor associated with each of the service users Ua is worn by the service user Ua. In the present embodiment, the processor 11 uses the communication interface 14 to acquire, from the relay terminal 3, an output signal from the temperature sensor provided close to the odor sensor. In a case where a temperature indicated by the output signal from the temperature sensor is equal to or higher than a preset threshold, the processor 11 determines that the odor sensor is "worn". In a case where the temperature indicated by the output signal from the temperature sensor is lower than the preset threshold, the processor 11 determines that the odor sensors is "not worn". In the present embodiment, the preset threshold is set to be 36.0°C by the training data generation program P1.

In a case where the determination "worn" is made in the wear determination process M11, the first acquisition process M12 is carried out. In a case where the determination "not worn" is made, the wear determination process M11 is repeatedly carried out. Thus, the processor 11 does not acquire an output signal from the odor sensor during a period during which each of the service users Ua does not wear the sensor device 4. This allows noise-reduced training data for generation of a model with high inference accuracy to be generated through the training data generation method M1.

The first acquisition process M12 is a process of acquiring the output signal from the odor sensor associated with each of the service users Ua together with the ID of the service user Ua. In the present embodiment, the processor 11 uses the communication interface 14 to acquire, from the relay terminal 3, the output signal from the odor sensor associated with the service user Ua, together with the ID of the service user Ua.

The processor 11 may acquire the output signal from each of the odor sensors in the first acquisition process M12 irrespective of whether or not the odor sensor has been, in the wear determination process M11, determined to be worn by the service user Ua. However, the present invention is not limited to this. For example, in the present embodiment, the processor 11 acquires, in the first acquisition process M12, only output signals from the odor sensors that have been, in the wear determination process M11, determined to be worn by the service users Ua.

The excretion determination process M13 is a process of determining whether or not each of the service users Ua has excreted, in view of the output signal from the odor sensor associated with the service user Ua. In the present embodiment, the processor 11 determines whether or not the service user Ua has excreted, in view of the output signal from the odor sensor associated with the service user Ua, the output signal having been acquired in the first acquisition process M12. In a case where the determination "excreted" is made in the excretion determination process M13, the notification process M14 is carried out.

The notification process M14 is a process of notifying each of the notification terminals 2 used by the service providers Ub that the excretion of each of the service users Ua has been detected, without the type of the excretion specified. In the present embodiment, the processor 11 uses the communication interface 14 to transmit, to the notification terminal 2, the notification screen σ1a indicative of the detection of the excretion of the service user Ua.

The notification terminal 2 displays, on the display thereof, the notification screen o1a (see Fig. 5) indicative of the detection of the excretion of the service user Ua. The service provider Ub taps the confirmation button on the notification screen σ1a and then treats the excretion of the service user Ua. In a case where the confirmation button on the notification screen o1a is tapped, the notification terminal 2 displays, on the display thereof, the input screen o1b (see Fig. 5) for input of the type of the excretion of the service user Ua. After the service provider Ub has treated the excretion of the service user Ua, the service provider Ub inputs the type of the excretion of the service user Ua with use of the input screen σ1b. In the present embodiment, in a case where the excreta in the undergarments of the service user Ua is a stool or urine, the service provider Ub selects the "stool" or the "urine", respectively, and, in a case where the excreta is absent in the undergarments, the service provider Ub selects "no stool or urine". In the present embodiment, in a case where the "no stool or urine" is selected, the processor 11 determines that the type of the excretion is flatus. After the input of the type of the excretion of the service user Ua with use of the input screen o1b, the notification terminal 2 transmits, to the main device 1, the type information indicative of the type inputted.

The second acquisition process M15 is a process of acquiring the type information indicative of a type of the excretion of each of the service users Ua. In the present embodiment, the processor 11 uses the communication interface 14 to acquire, from each of the notification terminals 2, the type information indicative of the type of the excretion of the service user Ua, the type information having been inputted by the service provider Ub with use of the input screen o1b.

The training data generation process M16 is a process of generating training data including (i) the output signal from the odor sensor associated with each of the service users Ua and (ii) type information indicative of the type of the excretion of the service user Ua, the type having been determined by the service provider Ub. In the present embodiment, the processor 11 generates the training data D including (i) the output signal that has been acquired in the first acquisition process M12 from each of the odor sensors that have been, in the wear determination process M11, determined to be worn by the service users Ua and (ii) the type information that has been acquired in the second acquisition process M15. In the training data D generated in the present embodiment, the output signal from each of the odor sensors that has been acquired during a predetermined period set with reference to the time point when the determination "excreted" had been made in the excretion determination process M13 is associated with the type information that has been acquired in the second acquisition process M15.

The training data D generated is associated with each of the IDs of the service users Ua that have been acquired in the first acquisition process M12 and is stored in the secondary memory 13.

### (Carrying out of Model Generation Method)

After the end of the tentative operation period, the main device 1 carries out the model generation method M2. Fig. 6 is a flowchart illustrating the flow of the model generation method M2.

As illustrated in Fig. 6, the model generation method M2 includes a model generation process M21. The model generation process M21 is a process of generating, through machine learning using the training data D generated in the tentative operation period, the model M into which the sensor information indicative of the output signal from the odor sensor is to be inputted and from which the type information indicative of the type of the excretion is to be outputted. In the present embodiment, the processor 11 generates, through machine learning using the training data D generated in the tentative operation period, the model M into which the sensor information indicative of the output signal from the odor sensor is to be inputted and from which the type information indicative of the type of the excretion is to be outputted.

Note that, in the model generation process M21, as the model M, the processor 11 may generate a general model applied to unspecific service users Ua or may generate special models each applied to the corresponding one of specific service users Ua. In the case of the general model, the processor 11 generates the model M through machine learning using all the pieces of the training data D generated in the tentative operation period. In the case of the special model, the processor 11 generates the model M corresponding to each of the service users Ua through machine learning using the training data D associated with the service user Ua, among all the pieces of the training data D generated in the tentative operation period. In the case of the special model, when the type of the excretion of each of the service users Ua is inferred, the model M corresponding to the service user Ua is used. This makes it possible to provide an accurate inference result.

### (Operation of Main device in Actual Operation Period)

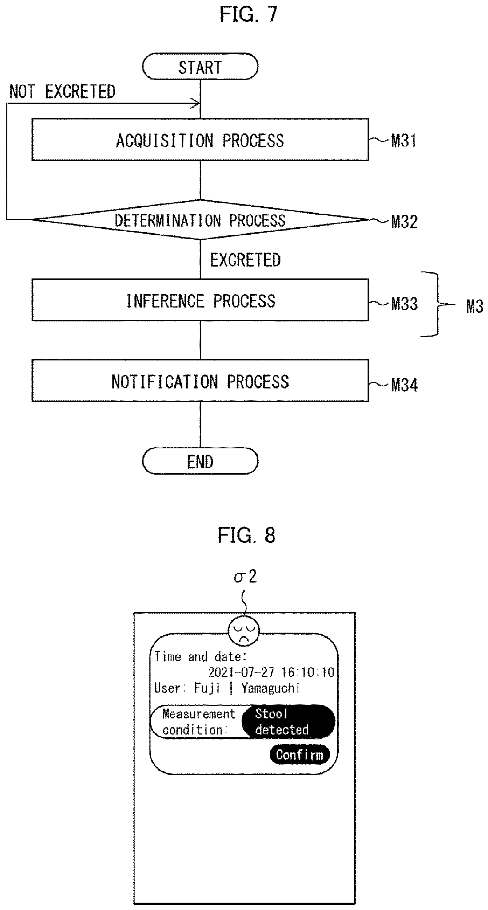

With reference to Figs. 7 and 8, the following description will discuss an operation performed by the main device 1 in the actual operation period. Fig. 7 is a flowchart illustrating a flow of the operation performed by the main device 1 in the actual operation period. Fig. 8 is a view illustrating an example of the notification screen o2 displayed on the notification terminal 2 in the actual operation period.

In the actual operation period, the main device 1 carries out an acquisition process M31, a determination process M32, an inference process M33, and a notification process M34 which are illustrated in Fig. 7. The inference process M33 constitutes the inference method M3.

The acquisition process M31 is a process of acquiring an output signal from the odor sensor associated with each of the service users Ua, together with the ID of the service user Ua. In the present embodiment, the processor 11 uses the communication interface 14 to acquire, from the relay terminal 3, the output signal from the odor sensor associated with the service user Ua, together with the ID of the service user Ua.

The determination process M32 is a process of determining whether or not each of the service users Ua has excreted, in view of the output signal from the odor sensor associated with the service user Ua. In the present embodiment, the processor 11 determines whether or not the service user Ua has excreted, in view of the output signal from the odor sensor associated with the service user Ua, the output signal having been acquired in the acquisition process M31. In a case where the determination "excreted" is made in the determination process M32, the inference process M33 is carried out.

The inference process M33 is a process of inferring the type of the excretion of each of the service users Ua in view of an output signal from the odor sensor associated with the service user Ua with use of the model M generated after the end of the tentative operation period and before the start of the actual operation period. In the present embodiment, the processor 11 infers the type of the excretion of each of the service users Ua in view of an output signal from the odor sensor associated with the service user Ua with use of the model M generated after the end of the tentative operation period and before the start of the actual operation period.

Note that, in the inference process M33, the processor 11 may make inference of the type of the excretion of the service user Ua with use of a general model M applied to the unspecific service users Ua or may make the inference with use of special models M each applied to the corresponding one of the specific service users Ua. In the case of the special models M, it is possible to provide an accurate inference result.

The notification process M34 is a process of notifying the notification terminals 2 used by the service providers Ub that the excretion of each of the service users Ua has been detected, with the type of the excretion specified. In the present embodiment, the processor 11 uses the communication interface 14 to transmit, to each of the notification terminals 2, the notification screen o2 which is indicative of the detection of the excretion of each of the service users Ua and which includes the inference result having been acquired in the inference process M33.

The notification terminal 2 displays, on the display thereof, the notification screen o2 (see Fig. 8) which is indicative of detection of certain types of the excretion of each of the service users Ua and which includes the inference result having been acquired in the inference process M33. This allows the service providers Ub to be informed of the excretion of the service user Ua together with the type of the excretion.

### (Variation)

The operation of the main device 1 in the actual operation period, in the present invention is not limited to that of the embodiment described above. For example, in the actual operation period, the processor 11 may display, on each of the displays, the input screen for input of the type of the excretion also in the notification process M34 like in the notification process M14. Further, after the notification process M34, the processor 11 may acquire the type of the excretion inputted by the service provider Ub and perform (i) generation of training data including the sensor information and the type information and (ii) relearning of the model using the training data generated. In such a variation, the processor 11 may subject the general model applied to the unspecific service users Ua to relearning using training data for each of the specific service users Ua. Such a variation makes it possible to generate, through the relearning, a model with high inference accuracy for specific service users Ua who have not undergone the tentative operation period, while applying the inference process M33 in the actual operation period also to the specific service users Ua.

In the actual operation period, the processor 11 may determine whether or not each of the odor sensors is worn by a service user Ua other than the service user Ua associated with the odor sensor. In such a variation, in a case where a concordance rate (correct answer rate) between the inference result made in the inference process M33 and the type of the excretion inputted by the service provider Ub is lower than a preset threshold, the processor 11 may determine that the odor sensor is worn by the service user Ua other than the service user Ua associated with the odor sensor.

In the present invention, the sub-sensor is not limited to the temperature sensor. The sub-sensor can be any sensor that outputs signals differing depending on whether or not the sensor device 4 is worn by the service user Ua. Examples of such a sub-sensor include a temperature sensor and a proximity sensor. Examples of the proximity sensor include a capacitive proximity sensor.

### (Recap)

A training data generation device in accordance with Aspect 1 of the present invention includes at least one processor, the at least one processor being configured to carry out: a wear determination process of determining, in view of an output signal from a sub-sensor provided close to an odor sensor associated with a service user, whether or not the odor sensor is worn by the service user; a first acquisition process of acquiring an output signal from the odor sensor; a second acquisition process of acquiring type information indicative of a type of excretion of the service user, the type having being determined by a service provider; and a training data generation process of generating training data which includes sensor information indicative of the output signal having been acquired in the first acquisition process from the odor sensor that has been, in the wear determination process, determined to be worn by the service user and which includes the type information having been acquired in the second acquisition process.

The training data generation device in accordance with Aspect 2 of the present invention is, in Aspect 1, arranged such that the at least one processor further carries out a notification process of notifying a notification terminal used by the service provider that the excretion of the service user has been detected; and the type information is inputted by the service provider with use of the notification terminal.

The training data generation device in accordance with Aspect 3 of the present invention is, in Aspect 1 or 2, arranged such that the sensor information is image data including a graph indicative of the output signal from the odor sensor.

A training data generation method in accordance with Aspect 4 of the present invention includes: carrying out, with use of at least one processor, a wear determination process of determining, in view of an output signal from a sub-sensor provided close to an odor sensor associated with a service user, whether or not the odor sensor is worn by the service user; carrying out, with use of the at least one processor, a first acquisition process of acquiring an output signal from the odor sensor; carrying out, with use of the at least one processor, a second acquisition process of acquiring type information indicative of a type of excretion of the service user, the type having being determined by a service provider; and carrying out, with use of the at least one processor, a training data generation process of generating training data which includes sensor information indicative of the output signal having been acquired in the first acquisition process from the odor sensor that has been, in the wear determination process, determined to be worn by the service user and which includes the type information having been acquired in the second acquisition process.

A model generation device in accordance with Aspect 5 of the present invention includes at least one processor, the at least one processor being configured to carry out a model generation process of generating, through machine learning using training data generated by the training data generation device in accordance with any one of Aspects 1 to 3, a model into which sensor information indicative of an output signal from an odor sensor is to be inputted and from which type information indicative of a type of excretion is to be outputted.

An inference device in accordance with Aspect 6 of the present invention includes at least one processor, the at least one processor being configured to carry out an inference process of inferring, with use of a model generated by the model generation device in accordance with Aspect 5, a type of excretion of a service user in view of an output signal from an odor sensor associated with the service user.

A program in accordance with Aspect 7 of the present invention is a program for causing a computer to function as the training data generation device in accordance with any one of Aspects 1 to 3.

A model generation device in accordance with Aspect 8 of the present invention includes at least one processor, the at least one processor being configured to carry out a model generation process of generating, through machine learning using training data, a model into which sensor information indicative of an output signal from an odor sensor is to be inputted and from which type information indicative of a type of excretion is to be outputted, the training data including sensor information indicative of an output signal from an odor sensor that is associated with a service user and that has been determined to be worn by the service user and type information indicative of a type of excretion of the service user, the type having been determined by a service provider.

A model generation device in accordance with Aspect 9 of the present invention is, in Aspect 8, arranged such that the service user includes a plurality of service users, and, in the model generation process, the at least one processor generates, through machine learning using training data, models into each of which sensor information indicative of an output signal from an odor sensor is to be inputted, from each of which type information indicative of a type of excretion is to be outputted, and each of which corresponds to a corresponding one of the plurality of service users, the training data including sensor information indicative of an output signal from an odor sensor that is associated with the corresponding one of the plurality of service users and that has been determined to be worn by the corresponding one of the plurality of service users and type information indicative of a type of excretion of the corresponding one of the plurality of service users, the type having been determined by a service provider.

A model generation device in accordance with Aspect 10 of the present invention is, in Aspect 8 or 9, arranged such that the sensor information is image data including a graph indicative of the output signal from the odor sensor.

A model generation device in accordance with Aspect 11 of the present invention is, in any one of Aspects 8 to 10, arranged such that, the at least one processor being configured to further carry out: a wear determination process of determining, in view of an output signal from a sub-sensor provided close to an odor sensor associated with a service user, whether or not the odor sensor is worn by the service user; a first acquisition process of acquiring an output signal from the odor sensor; a second acquisition process of acquiring type information indicative of a type of excretion of the service user, the type having being determined by a service provider; and a training data generation process of generating training data which includes sensor information indicative of the output signal having been acquired in the first acquisition process from the odor sensor that has been, in the wear determination process, determined to be worn by the service user and which includes the type information having been acquired in the second acquisition process, wherein, in the model generation process, the at least one processor generates, through machine learning using training data generated through the training data generation process, a model into which sensor information indicative of an output signal from an odor sensor is to be inputted and from which type information indicative of a type of excretion is to be outputted.

A model generation method in accordance with Aspect 12 of the present invention includes carrying out, with use of at least one processor, a model generation process of generating, through machine learning using training data, a model into which sensor information indicative of an output signal from an odor sensor is to be inputted and from which type information indicative of a type of excretion is to be outputted, the training data including sensor information indicative of an output signal from an odor sensor that is associated with a service user and that has been determined to be worn by the service user and type information indicative of a type of excretion of the service user, the type having been determined by a service provider.

An inference device in accordance with Aspect 13 of the present invention includes at least one processor, the at least one processor being configured to carry out an inference process of inferring, in view of an output signal from an odor sensor associated with a service user, a type of excretion of the service user with use of a model into which sensor information indicative of an output signal from an odor sensor is to be inputted and from which type information indicative of a type of excretion is to be outputted.

An inference device in accordance with Aspect 14 of the present invention is, in Aspect 13, arranged such that, in the inference process, the at least one processor infers, in view of an output signal from an odor sensor associated with a service user, a type of excretion of the service user with use of a model into which sensor information indicative of an output signal from an odor sensor is to be inputted, from which type information indicative of a type of excretion is to be outputted, and which corresponds to the service user.

An inference device in accordance with Aspect 15 of the present invention is arranged, in Aspect 13 or 14, such that the sensor information is image data including a graph indicative of the output signal from the odor sensor.

An inference device in accordance with Aspect 16 of the present invention is, in any one of Aspects 13 to 15, arranged such that the at least one processor being configured to further carry out a model generation process of generating, through machine learning using training data, a model into which sensor information indicative of an output signal from an odor sensor is to be inputted and from which type information indicative of a type of excretion is to be outputted, the training data including sensor information indicative of an output signal from an odor sensor that is associated with a service user and that has been determined to be worn by the service user and type information indicative of a type of excretion of the service user, the type having been determined by a service provider, wherein, in the inference process, the at least one processor infers, with use of a model generated through the model generation process, a type of excretion of a service user in view of an output signal from an odor sensor associated with the service user.

An inference device in accordance with Aspect 17 of the present invention is, in Aspect 16, arranged such that, the at least one processor being configured to further carry out: a wear determination process of determining, in view of an output signal from a sub-sensor provided close to an odor sensor associated with a service user, whether or not the odor sensor is worn by the service user; a first acquisition process of acquiring an output signal from the odor sensor; a second acquisition process of acquiring type information indicative of a type of excretion of the service user, the type having being determined by a service provider; and a training data generation process of generating training data which includes sensor information indicative of the output signal having been acquired in the first acquisition process from the odor sensor that has been, in the wear determination process, determined to be worn by the service user and which includes the type information having been acquired in the second acquisition process, wherein, in the model generation process, the at least one processor generates, through machine learning using training data generated through the training data generation process, a model into which sensor information indicative of an output signal from an odor sensor is to be inputted and from which type information indicative of a type of excretion is to be outputted.

An inference method in accordance with Aspect 18 of the present invention includes carrying out, with use of at least one processor, an inference process of inferring, in view of an output signal from an odor sensor associated with a service user, a type of excretion of the service user with use of a model into which sensor information indicative of an output signal from an odor sensor is to be inputted and from which type information indicative of a type of excretion is to be outputted.

### (Supplementary note)

The present invention is not limited to the embodiments above, but can be altered by a skilled person in the art within the scope of the claims. The present disclosure also encompasses, in its technical scope, any embodiment derived by combining technical means disclosed in differing embodiments as appropriate.

## Claims

1. A training data generation device comprising at least one processor, the at least one processor being configured to carry out:
- a wear determination process of determining, in view of an output signal from a sub-sensor provided close to an odor sensor associated with a service user, whether or not the odor sensor is worn by the service user;
- a first acquisition process of acquiring an output signal from the odor sensor;
- a second acquisition process of acquiring type information indicative of a type of excretion of the service user, the type having being determined by a service provider; and
- a training data generation process of generating training data which includes sensor information indicative of the output signal having been acquired in the first acquisition process from the odor sensor that has been, in the wear determination process, determined to be worn by the service user and which includes the type information having been acquired in the second acquisition process.

2. The training data generation device according to claim 1, wherein:
- the at least one processor further carries out a notification process of notifying a notification terminal used by the service provider that the excretion of the service user has been detected; and
- the type information is inputted by the service provider with use of the notification terminal.

3. The training data generation device according to claim 1, wherein the sensor information is image data including a graph indicative of the output signal from the odor sensor.

4. A training data generation method using at least one processor, the method comprising:
- carrying out, with use of the at least one processor, a wear determination process of determining, in view of an output signal from a sub-sensor provided close to an odor sensor associated with a service user, whether or not the odor sensor is worn by the service user;
- carrying out, with use of the at least one processor, a first acquisition process of acquiring an output signal from the odor sensor;
- carrying out, with use of the at least one processor, a second acquisition process of acquiring type information indicative of a type of excretion of the service user, the type having being determined by a service provider; and
- carrying out, with use of the at least one processor, a training data generation process of generating training data which includes sensor information indicative of the output signal having been acquired in the first acquisition process from the odor sensor that has been, in the wear determination process, determined to be worn by the service user and which includes the type information having been acquired in the second acquisition process.

5. A model generation device comprising at least one processor, the at least one processor being configured to carry out a model generation process of generating, through machine learning using training data generated by the training data generation device according to any one of claims 1 to 3, a model into which sensor information indicative of an output signal from an odor sensor is to be inputted and from which type information indicative of a type of excretion is to be outputted.

6. An inference device comprising at least one processor, the at least one processor being configured to carry out an inference process of inferring, with use of a model generated by the model generation device according to claim 5, a type of excretion of a service user in view of an output signal from an odor sensor associated with the service user.

7. A program for causing a computer to function as the training data generation device according to any one of claims 1 to 3.
